# EUROPEAN PATENT APPLICATION

(11) **EP 2 777 580 A2**
(43) Date of publication of application: **17.09.2014**
(21) Application number: 14158043.1
(22) Date of filing: 06.03.2014
(51) Int. Cl.: A61B 18/12

(54) **Bipolar gas plasma coagulation nozzle**

(30) Priority: 15.03.2013 US 201361789168 P; 03.02.2014 US 201414171018
(71) Applicant: Covidien LP, Mansfield, MA 02048 (US)
(72) Inventor: Wan, Logan C., Louisville, Colorado 80027 (US)
(74) Representative: HOFFMANN EITLE

(57) **Abstract**

A plasma instrument is disclosed. The plasma instrument includes an elongated body defining a first lumen therethrough, the lumen being in fluid communication with an ionizable media source; an applicator tip coupled to a distal end of the elongated body and disposed within the first lumen, the applicator tip defining a second lumen in fluid communication with the first lumen; a first electrode disposed on an outer surface of the applicator tip; and a second electrode disposed within at least one of the first lumen or the second lumen, wherein the first and second electrodes are configured to be energized to ignite ionizable media supplied by the ionizable media source.

## Description

### BACKGROUND

### Technical Field

The present disclosure relates to plasma devices and processes for surface processing and tissue treatment. More particularly, the disclosure relates to a bipolar coagulation handpiece for generating chemically reactive, plasma-generated species.

### Background of Related Art

Electrical discharges in dense media, such as liquids and gases at or near atmospheric pressure, can, under appropriate conditions, result in plasma formation. Plasmas have the unique ability to create large amounts of chemical species, such as ions, radicals, electrons, excited-state (e.g., metastable) species, molecular fragments, photons, and the like. The plasma species may be generated in a variety of internal energy states or external kinetic energy distributions by tailoring plasma electron temperature and electron density. In addition, adjusting spatial, temporal and temperature properties of the plasma creates specific changes to the material being irradiated by the plasma species and associated photon fluxes. Plasmas are also capable of generating photons including energetic ultraviolet photons that have sufficient energy to initiate photochemical and photocatalytic reaction paths in biological and other materials that are irradiated by the plasma photons.

### SUMMARY

Plasmas have broad applicability and provide alternative solutions to industrial, scientific and medical needs, especially workpiece (e.g, tissue) surface treatment at any temperature range. Plasmas may be delivered to the workpiece, thereby affecting multiple changes in the properties of materials upon which the plasmas impinge. Plasmas have the unique ability to create large fluxes of radiation (e.g., ultraviolet), ions, photons, electrons and other excited-state (e.g., metastable) species which are suitable for performing material property changes with high spatial, material selectivity, and temporal control. Plasmas may also remove a distinct upper layer of a workpiece with little or no effect on a separate underlayer of the workpiece or it may be used to selectively remove a particular tissue from a mixed tissue region or selectively remove a tissue with minimal effect to adjacent organs of different tissue type.

The plasma species are capable of modifying the chemical nature of tissue surfaces by breaking chemical bonds, substituting or replacing surface-terminating species (e.g., surface functionalization) through volatilization, gasification or dissolution of surface materials (e.g., etching). With proper techniques, material choices and conditions, one can remove one type of tissue entirely without affecting a nearby different type of tissue. Controlling plasma conditions and parameters (including S-parameters, V, I, Θ, and the like) allows for the selection of a set of specific particles, which, in turn, allows for selection of chemical pathways for material removal or modification as well as selectivity of removal of desired tissue type.

According to one embodiment, the present disclosure provides a plasma instrument. The plasma instrument includes an elongated body defining a first lumen therethrough, the lumen being in fluid communication with an ionizable media source; an applicator tip coupled to a distal end of the elongated body and disposed within the first lumen, the applicator tip defining a second lumen in fluid communication with the first lumen; a first electrode disposed on an outer surface of the applicator tip; and a second electrode disposed within at least one of the first lumen or the second lumen, wherein the first and second electrodes are configured to be energized to ignite ionizable media supplied by the ionizable media source.

According to another aspect of the above embodiment, the first electrode includes at least one of a plurality of concentric rings, a spiral, a plurality of interconnected strips, or a plurality of plates.

According to another aspect of the above embodiment, the elongated body includes a shaft housing enclosing a return lead coupled to the first electrode.

According to another aspect of the above embodiment, the shaft housing is flexible and the return lead is selectively tensionable to articulate the elongated body from a first generally relaxed position wherein proximal and distal portions of the elongated body are substantially aligned with a longitudinal axis defined by the elongated body to a second retracted position wherein the distal portion of the elongated body deflects from the longitudinal axis at a desired angle.

According to another aspect of the above embodiment, the elongated body includes shaft housing having a conductive sheath disposed therein.

According to another aspect of the above embodiment, the conductive sheath is in contact with the first electrode.

According to another aspect of the above embodiment, the elongated body includes an insulative sheath disposed within the shaft housing such that the conductive sheath is disposed between the shaft housing and the insulative sheath.

According to another aspect of the above embodiment, the first electrode includes an insulative layer and is supported within at least one of the first lumen or the second lumen by a spacer.

According to another embodiment, the present disclosure provides a plasma system. The plasma system includes: an electrosurgical generator; an ionizable media source; and a plasma instrument. The plasma instrument includes: an elongated body defining a first lumen therethrough, the lumen being in fluid communication with the ionizable media source; an applicator tip coupled to a distal end of the elongated body and disposed within the first lumen, the applicator tip defining a second lumen in fluid communication with the first lumen; a first electrode disposed on an outer surface of the applicator tip; and a second electrode disposed within at least one of the first lumen or the second lumen, wherein the first and second electrodes are coupled to the electrosurgical generator. The system also includes a return electrode pad configured to electrically couple to a patient; and a polarization controller electrically coupled to the return electrode pad, the polarization controller configured to adjust conductive coupling of the return electrode pad to the electrosurgical generator.

According to another aspect of the above embodiment, the polarization controller includes a variable resistance.

According to another aspect of the above embodiment, the variable resistance includes a plurality of resistors coupled to a plurality of switching elements configured to switch the plurality of resistors into the variable resistance.

According to another aspect of the above embodiment, the variable resistance includes a variable potentiometer controllable by an electromechanical actuator.

According to another aspect of the above embodiment, the variable resistance includes a voltage-controlled resistance selected from the group consisting of a transistor, a PIN diode, and combinations thereof.

According to another aspect of the above embodiment, at least one of the electrosurgical generator or the plasma instrument includes controls for adjusting resistance of the polarization controller.

According to another embodiment, the present disclosure provides a method. The method includes: supplying ionizable media to a plasma instrument; igniting the ionizable media at the plasma instrument. The plasma instrument includes: an elongated body defining a first lumen therethrough, the lumen being in fluid communication with an ionizable media source; an applicator tip coupled to a distal end of the elongated body and disposed within the first lumen, the applicator tip defining a second lumen in fluid communication with the first lumen; a first electrode disposed on an outer surface of the applicator tip; and a second electrode disposed within at least one of the first lumen or the second lumen; and adjusting variable resistance of a polarization controller coupled to a return electrode pad to control a degree of polarization of the plasma effluent.

According to another aspect of the above embodiment, the adjusting of the variable resistance includes sliding a slidable switch disposed on the plasma instrument.

According to another aspect of the above embodiment, the adjusting of the variable resistance includes inputting a desired degree of polarization using a polarization scale displayed on a screen of the electrosurgical generator.

In embodiments of the various aspects, the applicator tip is formed from a dielectric material.

In embodiments of the various aspects, the applicator tip includes a proximal portion configured and dimensioned to be inserted into the distal end of the first lumen.

In embodiments of the various aspects, the applicator tip includes a distal portion disposed outside the first lumen. In an embodiment, the distal portion has a larger diameter than that of the first lumen such that the distal portion has a cross-section that is substantially similar to or larger than the cross-section of the elongated body.

In embodiments of the various aspects, the first electrode constitutes an outer electrode and the second electrode constitutes an inner electrode.

In embodiments of the various aspects, the first electrode is disposed on a proximal portion of the applicator tip that is disposed within the first lumen.

In embodiments of the various aspects, the first electrode includes one or more electrodes that are insulated from the second electrode by dielectric material of the applicator tip allowing for capacitive coupling between the first and second electrodes.

In embodiments of the various aspects, the applicator tip is removably coupled to the elongated body such as by way of a mechanical interconnection including, but not limited to, frictional-type mount, a bayonet mount using one or more bayonet lugs, a thread mount using a threadable connection and combinations thereof. The removable applicator tip allows for interchangeability and/or selection of an applicator tip.

In embodiments of the various aspects, a system is provided including the instrument as described above and a plurality of applicator tips having different designs for the first electrode, differently shaped second lumens for tailoring the size and/or shape of the plasma plume generated by the instrument different dielectric materials to tailor coupling between the first and second electrodes, or otherwise a plurality of exchangeable applicator tips.

In embodiments of the various aspects, the elongated body of the instrument includes an electrically conductive sheath such that when a proximal portion of the applicator tip is inserted in the first lumen, the first electrode is in contact with the conductive sheath to connect the first electrode to electrosurgical energy. In an embodiment, an insulative sheath extends only up to the proximal portion of the applicator tip, thereby insulating ionizable media within the first lumen from the first electrode until the applicator tip.

In embodiments of the various aspects, the applicator tip is removable from the first lumen, wherein the second electrode is disposed within the first lumen when the applicator tip is removed.

In embodiments of the various aspects, the applicator tip is removably inserted in the first lumen and the second lumen receives the second electrode therein.

In a further independent aspect of the present disclosure, an applicator tip is provided. The applicator tip is removably couplable to a distal end of an elongated body of a plasma instrument and disposed within a first lumen defined through the elongated body, the applicator tip defining a second lumen for fluid communication with the first lumen. The applicator tip includes a first electrode disposed on an outer surface of a body of the applicator tip. The first electrode is configured to be energized to ignite ionizable media supplied by an ionizable media source through the first and second lumens.

The applicator tip of this aspect may include any of the above described features of the applicator tip.

In an embodiment, the applicator tip body has a larger cross-section distal portion relative to a proximal portion for receipt in the first lumen.

In an embodiment, the first electrode is disposed on an outer surface of the proximal portion.

In an embodiment, the body is made of a dielectric material.

The configuration of the first electrode may be as described above or otherwise herein.

In another aspect, a system of such applicator tips is provided for removable coupling to the elongated body of the same plasma instrument, by insertion of a proximal portion of the body in the first lumen.

In embodiments of the various aspects, the applicator tips have a plurality of different electrode designs for the first electrode, have differently shaped second lumens for tailoring the size and/or shape of the plasma plume generated by the instrument, have different dielectric materials to tailor coupling between the first and electrode a second electrode of the instrument, or otherwise provide a plurality of exchangeable applicator tips.

### BRIEF DESCRIPTION OF THE DRAWINGS

The accompanying drawings, which are incorporated in and constitute a part of this specification, illustrate exemplary embodiments of the disclosure and, together with a general description of the disclosure given above, and the detailed description of the embodiments given below, serve to explain the principles of the disclosure, wherein:
Fig. 1 is a perspective diagram of a plasma system according to the present disclosure;
Fig. 2 is a front elevational view of one embodiment of an electrosurgical generator according to the present disclosure;
Fig. 3 is a schematic, block diagram of the embodiment of an electrosurgical generator of Fig. 2 according to the present disclosure;
Fig. 4A is a longitudinal cross-sectional, side view of a plasma instrument of Fig. 1 according to the present disclosure;
Fig. 4B is a partial cross-sectional, perspective view of an enlarged area 4B as indicated in Fig. 4A of an elongated body of the plasma instrument of Fig. 1 according to the present disclosure;
Figs. 5A-E are perspective views of multiple embodiments of an applicator tip of the plasma instrument of Fig. 1 according to the present disclosure;
Fig. 6A is a partial cross-sectional, perspective view of the distal portion of another embodiment of an elongated body of the plasma instrument of Fig. 1 according to the present disclosure;
Fig. 6B is a cross-sectional, front view the elongated body of the plasma instrument of Fig. 6A taken along section line 6B-6B according to the present disclosure; and
Fig. 7 is a schematic, block diagram of another embodiment of the plasma system of Fig. 1 according to the present disclosure.

### DETAILED DESCRIPTION

Plasmas may be generated using electrical energy that is delivered as either direct current (DC) electricity or alternating current (AC) electricity at frequencies from about 0.1 hertz (Hz) to about 100 gigahertz (GHz), including radio frequency ("RF", from about 0.1 MHz to about 100 MHz) and microwave ("MW", from about 0.1 GHz to about 100 GHz) bands, using appropriate generators, electrodes, and antennas. Choice of excitation frequency, the workpiece, as well as the electrical circuits that are used to deliver electrical energy to the workpiece affect many properties and requirements of the plasma. The performance of the plasma chemical generation, the delivery system and the design of the electrical excitation circuitry are interrelated -- as the choices of operating voltage, frequency and current levels (as well as phase) effect the electron temperature and electron density. Further, choices of electrical excitation and plasma device hardware also determine how a given plasma system responds dynamically to the introduction of new ingredients to the host plasma gas or liquid media.

Plasma beams may be used to coagulate, cauterize, or otherwise treat tissue through direct application of high-energy plasma. In particular, kinetic energy transfer from the plasma to the tissue causes healing, and thus, affects thermal coagulation of bleeding tissue. Plasma beam coagulation utilizes a handheld electrosurgical instrument having one or more electrodes energizable by an electrosurgical generator, which outputs a high-intensity electric field suitable for forming plasma using ionizable media (e.g., inert gas).

Plasma beam coagulation systems may be polarized or non-polarized. As used herein, the term "polarized" refers to plasma systems that include a return electrode disposed outside the instrument, that is coupled to a patient (e.g., outside the treatment site). During operation of polarized plasma, the instrument including one or more active electrodes comes into close proximity with the patient, the electric field intensity becomes sufficient to ionize the gas thereby forming plasma. Plasma provides a conductive path to the patient and without being limited by any particular theory, it is believed that the clinical effect is primarily effected by resistance heating of the patient tissue as the electrosurgical current passes through the patient and to the return electrode.

As used herein, the term "non-polarized" refers to plasma systems that include a handheld electrosurgical instrument having both an active and a return electrode. The system does not include a separate return electrode coupled to the patient, thus isolating the patient from the electrosurgical generator. Electrosurgical energy is provided by the generator and forms an electric field between the electrodes contained within the instrument. In this configuration plasma is generated within the instrument and is delivered to the patient as gas is pushed out of the instrument. Without being bound by any particular theory, it is believed that primary clinical effect in non-polarized system is due to the transfer of kinetic and thermal energy of the plasma.

Polarized plasma systems produce faster coagulation that non-polarized systems. However, speed and degree of coagulation is difficult to control using conventional electrosurgical generators, since specialized circuitry is required that can vary plasma intensity without extinguishing the plasma. Further, polarized electric fields produced between the instrument and tissue are attracted and/or deflected by the plasma beam, making it difficult to aim the beam. Non-polarized systems avoid the aiming difficulty of polarized systems, but are slower in producing desired tissue effects. Furthermore, such systems also rely on specialized direct current generators, which have no utility in other electrosurgical modalities.

The present disclosure provides for a bipolar plasma instrument (e.g., having both an active and a return electrode) and a hybrid polarization plasma system that can be operated in polarized, non-polarized and hybrid manner to overcome the drawbacks of polarized and non-polarized systems. The system includes an electrosurgical generator and an ionizable media source. The system further includes a plasma instrument having two or more electrodes (e.g., bipolar) coupled to the generator and the ionizable media source and a return electrode in contact with a patient that is also coupled to the generator via a polarization controller having variable resistance. The system includes controls disposed at the generator and/or the instrument for adjusting the resistance of the polarization controller to adjust the degree of polarization of the plasma generated by the instrument. Thus, using a bipolar plasma surgical instrument and varying the extent to which the patient is electrically-coupled to the generator via the return electrode, allows for varying the degree of polarization of the plasma beam (e.g., from purely polarized to purely non-polarized) and therebetween.

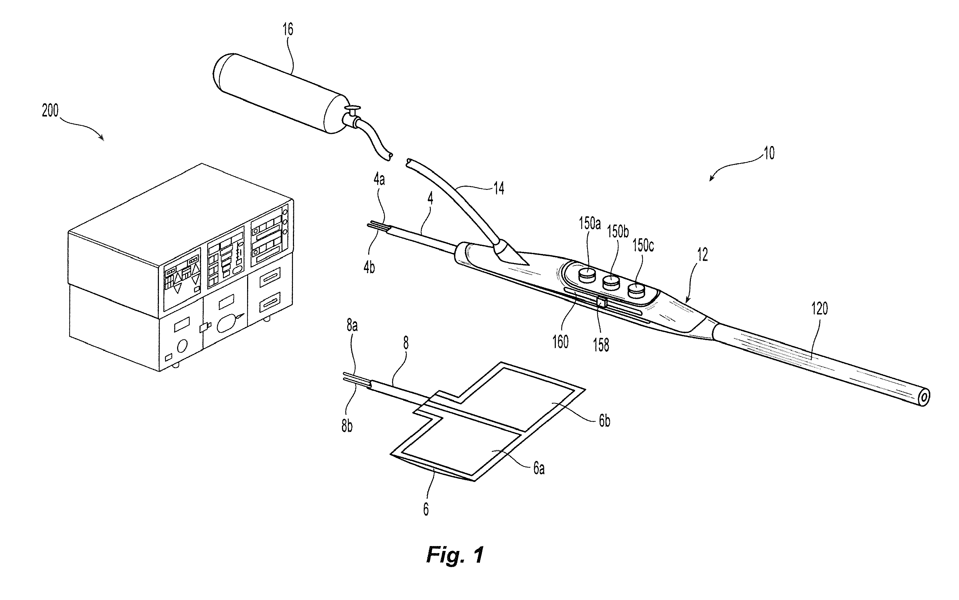

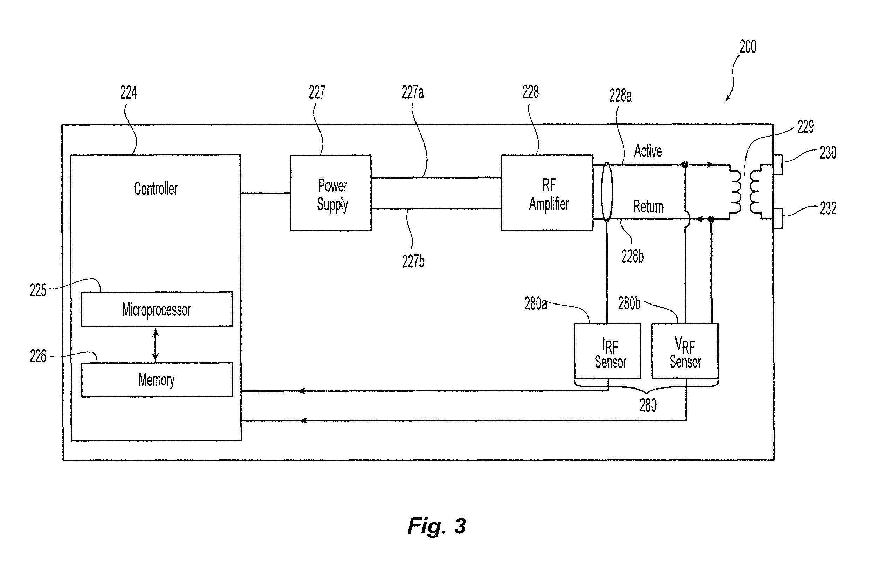

Referring initially to Fig. 1, a plasma system 10 is disclosed. The system 10 includes a plasma instrument 12 that is coupled to a generator 200, an ionizable media source 16 which may also include an optional precursor source (not shown). Generator 200 includes any suitable components for delivering power to the plasma instrument 12. More particularly, the generator 200 may be any radio frequency generator or other suitable power source capable of producing power to ignite the ionizable media to generate plasma. In embodiments, electrosurgical energy is supplied to the instrument 12 by the generator 200 via an instrument cable 4. The cable 4 includes a supply lead 4a connecting the instrument 12 to an active terminal 230 (Fig. 3) of the generator 200 and a return lead connecting the instrument 12 to a return terminal 232 (Fig. 3) of the generator 200. The plasma instrument 12 may be utilized as an electrosurgical pencil for application of plasma to tissue and the Generator 200 may be an electrosurgical generator that is adapted to supply the instrument 12 with electrical power at a frequency from about 100 kHz to about 4 MHz, in embodiments the frequency may range from about 200 kHz to about 3 MHz, in further embodiments the frequency may range from about 300 kHz to about 1 MHz.

The system 10 also includes one or more return electrode pads 6 that, in use, are disposed on a patient to minimize the chances of tissue damage by maximizing the overall contact area with the patient. The return electrode pad 6 may include two or more split electrodes 6a, 6b. The generator 200 may be configured to measure impedance between the split electrodes 6a, 6b to monitor tissue-to-patient contact to ensure that sufficient contact exists between the electrode pad 6 and the patient. The energy is returned to the generator 200 through the return electrode pad 6 via one or more return leads 8a, 8b, housed within a return pad cable 8 at the return terminal 232 (Fig. 3) of the generator 200. In particular, each of the return leads 8a, 8b is connected to one or more split electrodes 6a, 6b of the return electrode pad 6.

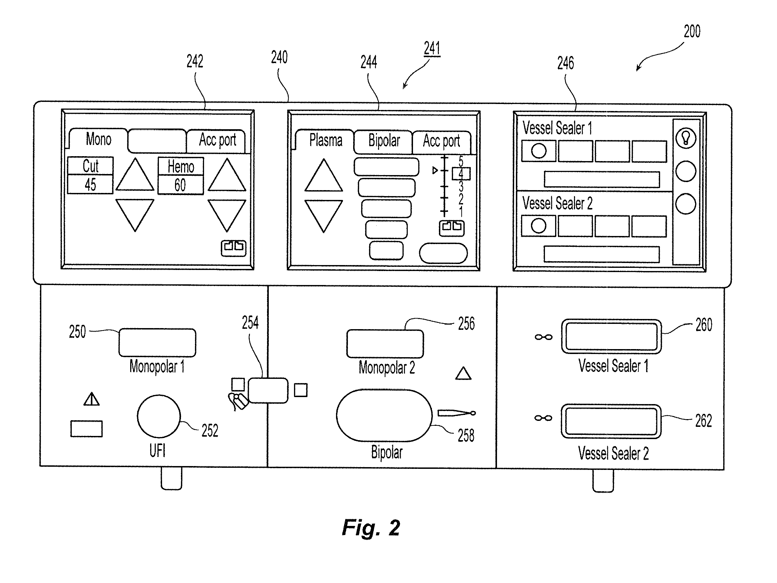

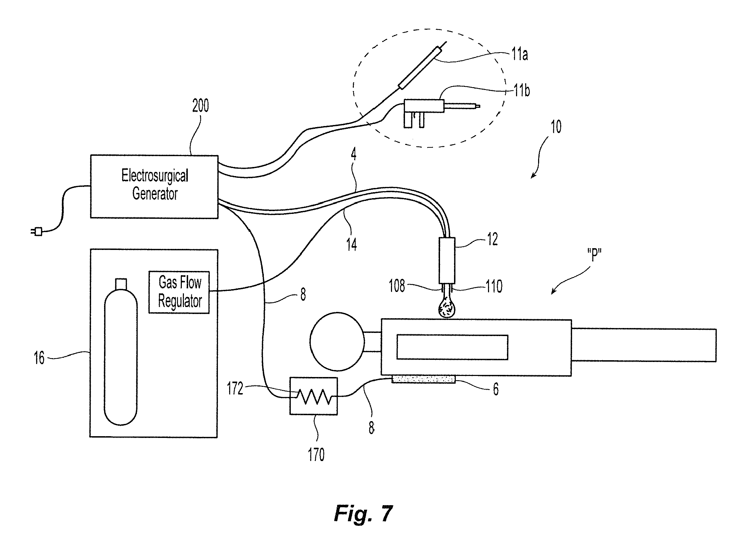

With reference to Fig. 2, a front face 240 of the generator 200 is shown. The generator 200 may be any suitable type (e.g., electrosurgical, microwave, etc.) and may include a plurality of connectors 250-262 to accommodate various types of electrosurgical instruments (e.g., electrosurgical forceps, electrosurgical pencils, ablation probes, etc.) in addition to the plasma instrument 12 as shown in Fig. 7.

The generator 200 includes a user interface 241 having one or more display screens 242, 244, 246 for providing the user with variety of output information (e.g., intensity settings, treatment complete indicators, etc.). Each of the screens 242, 244, 246 is associated with corresponding connector 250, 252, 254, 256, 258, 260, and 262. The generator 200 includes suitable input controls (e.g., buttons, activators, switches, touch screen, etc.) for controlling the generator 200. The display screens 242, 244, 246 are also configured as touch screens that display a corresponding menu for the electrosurgical instruments (e.g., plasma instrument 12, etc.). The user then adjusts inputs by simply touching corresponding menu options.

Screen 242 controls monopolar output and the devices connected to the connectors 250 and 252. Connector 250 is configured to couple to a monopolar electrosurgical instrument (e.g., electrosurgical pencil) and connector 252 is configured to couple to a foot switch (not shown). The foot switch provides for additional inputs (e.g., replicating inputs of the generator 200). Screen 244 controls monopolar, plasma and bipolar output and the devices connected to the connectors 256 and 258. Connector 256 is configured to couple to other monopolar instruments. Connector 258 is configured to couple to plasma instrument 12.

Connector 254 may be used to connect to one or more return electrode pads 6. The return electrode pad 6 is coupled to the generator 200 via the return pad cable 8, which is coupled to the connector 254 via a plug (not shown). The return electrode pad 6 is coupled to a polarization controller 170, which is in turn coupled to the connector 254 (as shown in Fig. 7), which is described in further detail below. Screen 246 controls plasma procedures performed by the plasma instrument 12 that may be plugged into the connectors 260 and 262.

Fig. 3 shows a schematic block diagram of the generator 200 configured to output electrosurgical energy. The generator 200 includes a controller 224, a power supply 227, and a radio-frequency (RF) amplifier 228. The power supply 227 may be a high voltage, DC power supply connected to an AC source (e.g., line voltage) and provides high voltage, DC power to the RF amplifier 228 via leads 227a and 227b, which then converts high voltage, DC power into treatment energy (e.g., electrosurgical or microwave) and delivers the energy to the active terminal 230. The energy is returned thereto via the return terminal 232. The active and return terminals 230 and 232 and coupled to the RF amplifier 228 through an isolation transformer 229. The RF amplifier 228 is configured to operate in a plurality of modes, during which the generator 200 outputs corresponding waveforms having specific duty cycles, peak voltages, crest factors, etc. It is envisioned that in other embodiments, the generator 200 may be based on other types of suitable power supply topologies.

The controller 224 includes a processor 225 operably connected to a memory 226, which may include transitory type memory (e.g., RAM) and/or non-transitory type memory (e.g., flash media, disk media, etc.). The processor 225 includes an output port that is operably connected to the power supply 227 and/or RF amplifier 228 allowing the processor 225 to control the output of the generator 200 according to either open and/or closed control loop schemes. A closed loop control scheme is a feedback control loop, in which a plurality of sensors measure a variety of tissue and energy properties (e.g., tissue impedance, tissue temperature, output power, current and/or voltage, etc.), and provide feedback to the controller 224. The controller 224 then signals the power supply 227 and/or RF amplifier 228, which adjusts the DC and/or power supply, respectively. Those skilled in the art will appreciate that the processor 225 may be substituted by using any logic processor (e.g., control circuit) adapted to perform the calculations and/or set of instructions described herein including, but not limited to, field programmable gate array, digital signal processor, and combinations thereof.

The generator 200 according to the present disclosure includes a plurality of sensors 280, e.g., an RF current sensor 280a, and an RF voltage sensor 280b. Various components of the generator 200, namely, the RF amplifier 228, the RF current and voltage sensors 280a and 280b, may be disposed on a printed circuit board (PCB). The RF current sensor 280a is coupled to the active terminal 230 and provides measurements of the RF current supplied by the RF amplifier 228. In embodiments the RF current sensor 280a may be coupled to the return terminal 232. The RF voltage sensor 280b is coupled to the active and return terminals 230 and 232 provides measurements of the RF voltage supplied by the RF amplifier 228. In embodiments, the RF current and voltage sensors 280a and 280b may be coupled to active and return leads 228a and 228b, which interconnect the active and return terminals 230 and 232 to the RF amplifier 228, respectively.

The RF current and voltage sensors 280a and 280b provide the sensed RF voltage and current signals, respectively, to the controller 224, which then may adjust output of the power supply 227 and/or the RF amplifier 228 in response to the sensed RF voltage and current signals. The controller 224 also receives input signals from the input controls of the generator 200 and/or the plasma instrument 12. The controller 224 utilizes the input signals to adjust the power output of the generator 200 and/or performs other control functions thereon.

With reference once again to Fig. 1, the system 10 provides a flow of plasma through the instrument 12 to a workpiece (e.g., tissue). Plasma feedstocks, which include ionizable media and optional precursor feedstocks, are supplied by the ionizable media source 16 to the plasma instrument 12. The ionizable media source 16 may include various flow sensors and controllers (e.g., valves, mass flow controllers, etc.) to control the flow of ionizable media to the instrument 12. During operation, the ionizable media and/or the precursor feedstock are provided to the plasma instrument 12 where the plasma feedstocks are ignited to form plasma effluent containing ions, radicals, photons from the specific excited species and metastables that carry internal energy to drive desired chemical reactions in the workpiece or at the surface thereof. The feedstocks may be mixed upstream from the ignition point or midstream thereof (e.g., at the ignition point) of the plasma effluent.

The ionizable media source 16 may include a storage tank, a pump, and/or flow meter (not explicitly shown). The ionizable media may be a liquid or a gas such as argon, helium, neon, krypton, xenon, radon, carbon dioxide, nitrogen, hydrogen, oxygen, etc. and their mixtures, and the like. These and other gases may be initially in a liquid form that is gasified during application. The precursor feedstock may be either in solid, gaseous or liquid form and may be mixed with the ionizable media in any state, such as solid, liquid (e.g., particulates or droplets), gas, and the combination thereof.

With continued reference to Fig. 1, the ionizable media source 16 may be coupled to the plasma instrument 12 via tubing 14. The tubing 14 may be fed from multiple sources of ionizible media and/or precursor feedstocks, which may combined into unified tubing to deliver a mixture of the ionizable media and the precursor feedstock to the instrument 12 at a proximal end thereof. This allows for the plasma feedstocks, e.g., the precursor feedstock and the ionizable gas, to be delivered to the plasma instrument 12 simultaneously prior to ignition of the mixture therein.

In another embodiment, the ionizable media and precursor feedstocks may be supplied at separate connections, such that the mixing of the feedstocks occurs within the plasma instrument 12 upstream from the ignition point such that the plasma feedstocks are mixed proximally of the ignition point.

In a further embodiment, the plasma feedstocks may be mixed midstream, e.g., at the ignition point or downstream of the plasma effluent, directly into the plasma. It is also envisioned that the ionizable media may be supplied to the instrument 12 proximally of the ignition point, while the precursor feedstocks are mixed therewith at the ignition point. In a further illustrative embodiment, the ionizable media may be ignited in an unmixed state and the precursors may be mixed directly into the ignited plasma. Prior to mixing, the plasma feedstocks may be ignited individually. The plasma feedstock may be supplied at a predetermined pressure to create a flow of the medium through the instrument 12, which aids in the reaction of the plasma feedstocks and produces a plasma effluent. The plasma according to the present disclosure may be generated at or near atmospheric pressure under normal atmospheric conditions.

In one embodiment, the precursors may be any chemical species capable of forming reactive species such as ions, electrons, excited-state (e.g., metastable) species, molecular fragments (e.g., radicals) and the like, when ignited by electrical energy from the Generator 200 or when undergoing collisions with particles (electrons, photons, or other energy-bearing species of limited and selective chemical reactivity) formed from ionizable media 16. More specifically, the precursors may include various reactive functional groups, such as acyl halide, alcohol, aldehyde, alkane, alkene, amide, amine, butyl, carboxlic, cyanate, isocyanate, ester, ether, ethyl, halide, haloalkane, hydroxyl, ketone, methyl, nitrate, nitro, nitrile, nitrite, nitroso, peroxide, hydroperoxide, oxygen, hydrogen, nitrogen, and combination thereof. In embodiments, the precursor feedstocks may be water, halogenoalkanes, such as dichloromethane, tricholoromethane, carbon tetrachloride, difluoromethane, trifluoromethane, carbon tetrafluoride, and the like; peroxides, such as hydrogen peroxide, acetone peroxide, benzoyl peroxide, and the like; alcohols, such as methanol, ethanol, isopropanol, ethylene glycol, propylene glycol, alkalines such as NaOH, KOH, amines, alkyls, alkenes, and the like. Such precursor feedstocks may be applied in substantially pure, mixed, or soluble form.

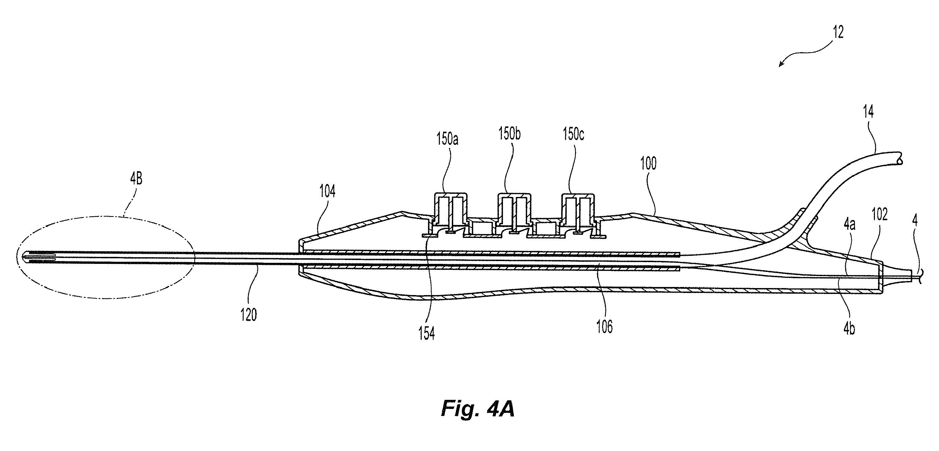

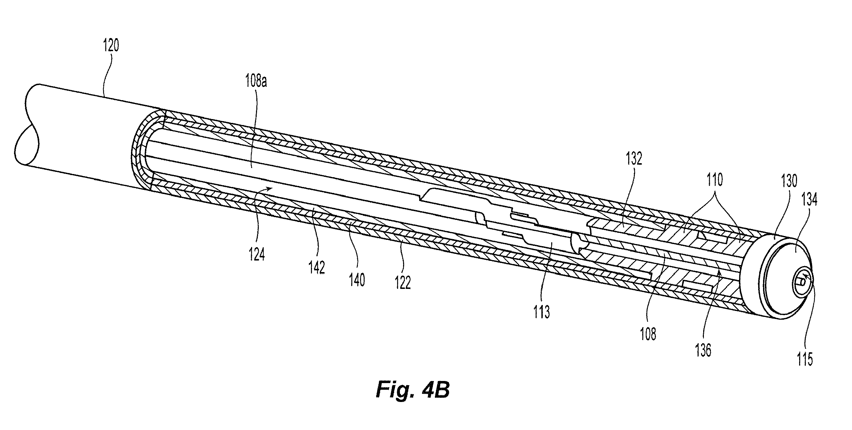

With reference to Figs. 1 and 4A-B, the instrument 12 includes a handle housing 100 having a proximal end 102 and a distal end 104. The housing 100 also includes a lumen 106 defined therein having a proximal end coupled to the gas tubing 14 from the ionizable media source 16 and a distal end terminating at the distal end 104 of the housing 100. The instrument 12 also includes an elongated body 120 having a shaft housing 122 defining a lumen 124 therethrough as shown in Fig. 4B. The shaft housing 122 may be rigid or flexible. In particular, the lumens 106 and 124 are in gaseous and/or fluid communication with the ionizable media source 16 allowing for the flow of ionizable media and precursor feedstocks to flow through the lumens 106 and 124.

With reference to Figs. 4A-B, conductors 4a, 4b are coupled to the electrodes 108 and 110, respectively. The conductors 4a, 4b extend through the housing 100 and shaft housing 122 of the elongated body 120 and are connected to the generator 200 via the cable 4. The cable 4 may include a plug (not shown) connecting the instrument 12 to the generator 200 at the connector 258. Each of the electrodes 108 and 110 is connected to the generator 200 and may therefore be energized by the generator 200 allowing the instrument 12 to operate in non-polarized manner as described in further detail below. The conductor 4a is coupled to the proximal end of the electrode 108. The conductor 4b may be a lead or a wire embedded in the shaft housing 122 and is coupled to the electrode 110 by exposing a distal portion of the conductor 4b as shown in Fig. 4B.

The shaft housing 122 may have a diameter from about 2 mm to about 20 mm allowing the instrument 12 to be inserted through operating ports of an endoscope or access ports in laparoscopic procedures as well as natural body orifices for application of the plasma effluent at the operating site during minimally invasive procedures.

The shaft housing 122 may be formed from any suitable dielectric material including thermoplastics, such as acrylics, celluloid, cellulose acetate, cyclic olefin copolymer, ethylene-vinyl acetate, fluoropolymers (e.g., polytetrafluoroethylene), ionomers, polyoxymethylene, polyacrylates, polyacrylonitrile, polyamide, polyamide-imide, polyaryletherketon, polybutadiene, polybutylene, polybutylene terephthalate, polycaprolactone, polychlorotrifluoroethylene, polyethylene terephthalate, polycyclohexylene dimethylene terephthalate, polycarbonate, polyhydroxyalkanoates, polyketones, polyester, polyethylene, polyetheretherketone, polyetherketoneketone, polyetherimide, polyethersulfone, chlorinated polyethylene, polyimide, polylactic acid, polymethylpentene, polyphenylene oxide, polyphenylene sulfide, polyphthalamide, polypropylene, polystyrene, polysulfone, polytrimethylene terephthalate, polyurethane, polyvinyl acetate, polyvinyl chloride, polyvinylidene chloride, styrene-acrylonitrile, and combinations thereof.

With reference to Fig. 4B, the instrument 12 further includes an applicator tip 130 coupled to the elongated body 120 at the distal end thereof. In embodiments, the applicator tip 130 is inserted into the distal end of the lumen 124. The tip 130 may be formed from any suitable dielectric materials including the thermoplastic materials described above if the temperature of the plasma is sufficiently low or any other suitable heat-resistant dielectric material, including ceramic materials. Suitable ceramic materials include, but are not limited to, metal oxide ceramics, non-oxide ceramics, ceramic composites, and combinations thereof. Suitable oxide ceramics include zirconium oxide, aluminum oxide, silica oxide, magnesium oxide, iron oxide, calcium oxide, yttrinum oxide, cerium oxide, alumina oxide, silicon oxide, calcium silicate, copper oxide, nickel oxide, praseodymium oxide, titanium oxide, erbium oxide, europium oxide, holmium oxide, chromium oxide, manganese oxide, vanadium oxide, cobalt oxide, neodymium oxide and combinations and composites thereof such as fiber composites, metal oxide composites, non-oxide composites, alumina/zirconia composites, and the like.

With reference to Fig. 4B, the applicator tip 130 includes a proximal portion 132 configured and dimensioned to be inserted into the distal end of the lumen 124. The applicator tip 130 also includes a distal portion 134 disposed outside the lumen 124. In embodiments, the distal portion 134 may have a larger diameter than that of the lumen 124 such that the distal portion 134 has a cross-section that is substantially similar to or larger than the cross-section of the shaft housing 122 to protect the shaft housing 122 from the plasma generated at the applicator tip 130.

The applicator tip 130 also includes a lumen 136 defined therethrough that is fluid communication with the lumen 124. The lumen 136 may have any suitable shape for tailoring the size and/or shape of the plasma plume generated by the instrument 12. In embodiments, the lumen 124 may also include one or more surfaces for further shaping (e.g., narrowing) the plasma plume prior to exiting the instrument 12.

With reference to Fig. 4B, the instrument 12 also includes two or more electrodes 108, 110 disposed within the applicator tip 130, shown as an inner and outer electrodes, respectively. The electrodes 108 and 110 may be formed from a conductive material including metals, such as stainless steel, copper, aluminium, tungsten, and combinations and alloys thereof. The electrodes 108 and 110 may have any suitable shape for conducting electrical energy and igniting the ionizable media including, but not limited to, rings, strips, needles, meshes, and the like. The electrodes 108 and 110 may also be disposed outside or within the lumen 124 for capacitive coupling with the ionizable media as described in further detail below. The ionizable media in conjunction with the optional precursor feedstocks is ignited by application of energy through the electrodes 108 and 110 to form a plasma plume exiting through an opening 115 of the applicator tip 130.

In embodiments, the electrode 108 may be configured as an inner electrode as shown in Fig. 4B. The electrode 108 may be enclosed in an insulative layer 108a and may be supported within the lumen using a spacer 113.

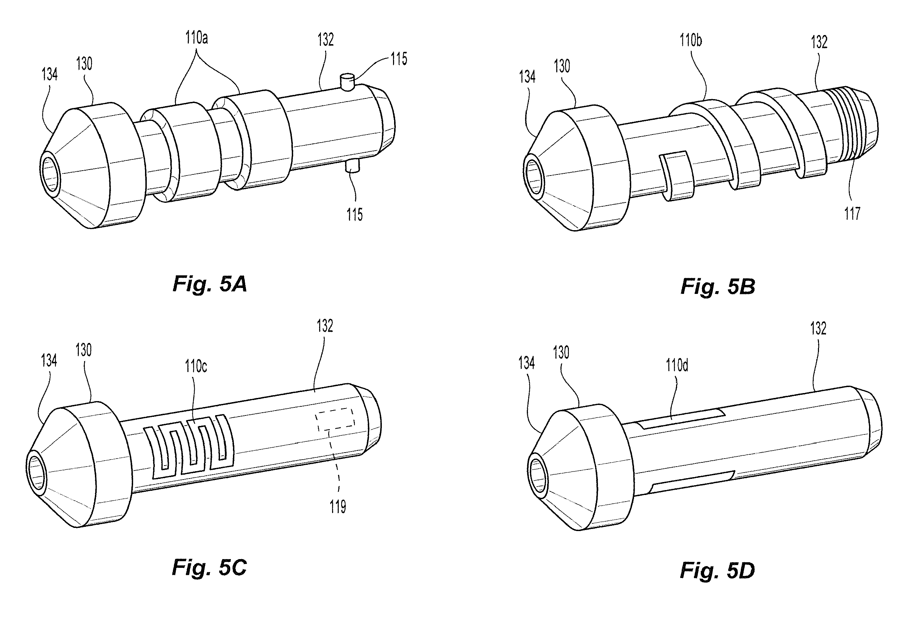

The electrode 110 is disposed on the proximal portion 132 of the applicator tip 130. The electrode 110 may include one or more electrodes that are insulated from the electrode 108 by the dielectric material of the applicator tip 130 allowing for capacitive coupling between the electrodes 108 and 110. The electrode 110 may include one or more concentric rings 110a as shown in Fig. 5A, a spiral 110b as shown in Fig. 5B, a plurality of interconnected strips 110c as shown in Fig. 5C, one or more plates 110d as shown in Fig. 5D.

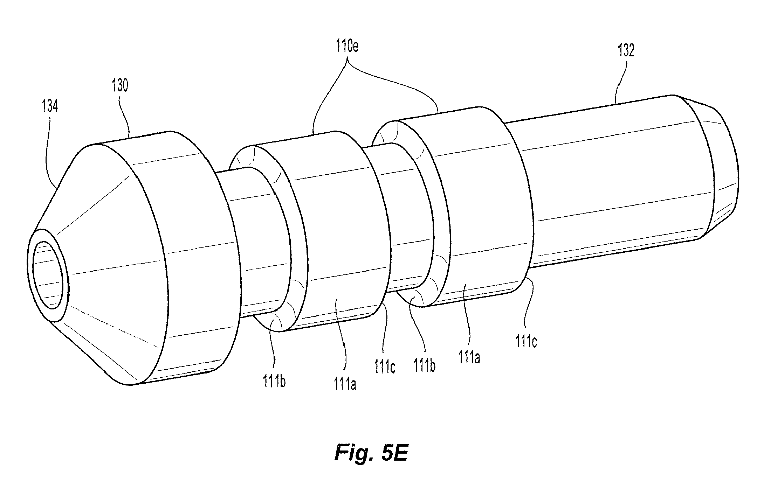

Fig. 5E shows another embodiment of one or more electrodes 110e having a substantially ring-like shape. The electrode 110e includes a raised surface 111a having a proximal and distal concave edges 111b, 111c. The concave shape of the edges 111b, 111c provides for a sharp transition between the edges 111b, 111c and the surface 111a. This transition provides for concentration of the electrical field generated between the electrodes 108 and 110e resulting in higher transfer of energy to the plasma.

The electrodes 110a-110e may be formed from any suitable conductive material (e.g., metals) using machining, forging, metal injection molding, and other suitable methods. The electrodes 110a-110e may then be bonded to the surface of the applicator tip 130 using adhesives or any other suitable methods.

In embodiments, the electrodes 110a-110e may be formed from conductive particles using various methods including, but not limited to, plasma deposition, atomic layer deposition, screen printing, spraying, painting and combinations thereof. The conductive particles may be applied to any desired structure for forming the electrode, e.g., ring-like structure of the electrode 110e including the raised surface 111a and concave edges 111b, 111c. In embodiments, the electrode structure may be formed from a dielectric material, e.g., the applicator tip 130. In further embodiments, inks including a plurality of conductive particles suspended in a solvent may be used to form the electrodes 110a-110e. After application, the solvent in the ink evaporates following application on the surface of the applicator tip 130 leaving behind a layer of conductive particles thereby forming the electrodes 110a-110e.

In embodiments, the applicator tip 130 may be removably coupled to the elongated body 120 using any suitable mechanical interconnection including, but not limited to frictional-type mount, a bayonet mount using one or more bayonet lugs 115 as shown in Fig. 5A, a thread mount using a threadable connection 117 as shown in Fig. 5B, and combinations thereof. The distal portion of the lumen 124 includes a corresponding connection, such as one or more slits for interfacing with the bayonet lugs 115, a threadable connection, and the like.

Removable applicator tips 130 allows for interchangeability and selection of the suitable applicator tip 130 for the application. Applicator tips 130 may have various electrode designs as described above with respect to Figs. 5A-5E, differently shaped lumen 136 for tailoring the size and/or shape of the plasma plume generated by the instrument 12. In embodiments, the applicator tips 130 may also be formed from different dielectric materials to tailor coupling between the electrodes 108, 110. Applicator tip 130 maybe a reusable component used to extend the life of instrument 12 in long procedures or across multiple procedures. Thus, a method of exchanging the applicator tips 130 is an important aspect in current market trends where remanufacture of instruments reduces consumer cost.

In further embodiments, the applicator tip 130 may include an identifier 119 as shown in Fig. 5C configured to store one or more values corresponding to properties of the applicator tip 130. The identifier 119 may be RFID, EEPROM or any other suitable storage medium accessible by the generator 200. Values stored in the identifier 119 may include, but are not limited to, electrode type/structure, dielectric material of the applicator tip 130, shape/structure of the lumen 136, serial number, and the like. In further embodiments, the storage medium (e.g., non-transitory) identifier 119 may be wholly or partially rewritable and may store usage data including sterilization counts, usage counts, time used and the like.

The generator 200 may include a corresponding reader/writer configured to interface with the identifier 119. The generator 200 may tailor its output based on the data stored in the identifier 119 as well as update the identifier 119 to reflect usage/sterilization data after the instrument 12 is used.

With reference to Fig. 4B, the instrument 12 also includes an electrically conductive sheath 140 disposed between the shaft housing 122 and an inner insulative sheath 142, which defines the lumen 124. The conductive sheath 140 is coupled to the conductor 4b and extends up to the proximal end of the shaft housing 122 such that when the proximal portion 132 of the applicator tip 130 is inserted, the electrodes 110 (e.g., electrodes 110e) are in contact with the conductive sheath 140. The insulative sheath 142 extends only up to the proximal portion 132 of the applicator tip 130, namely, the proximal end of the electrode 110, thus fully insulating the ionizable media within the lumen 124 from the electrodes 108, 110 until the applicator tip 130.

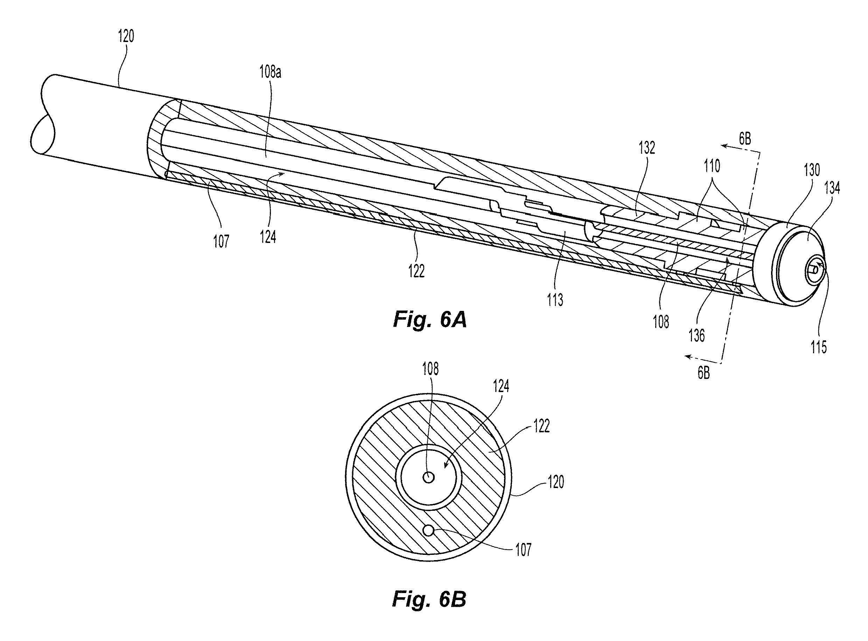

Figs. 6A-6B show another embodiment of the instrument 12 in which the shaft housing 122 is flexible and is configured for passive or controlled deflection. A pull-wire 107 or another suitable actuation mechanism extends from the handle housing 100 at the proximal end of the shaft housing 122. The pull-wire 107 is movable from a first generally relaxed position wherein the proximal and distal portions of the shaft housing 122 are substantially aligned with a longitudinal axis defined by the shaft to a second retracted or tensed position wherein the distal portion flexes (e.g., deflects) from the longitudinal axis at a desired angle. The pull-wire 107 may be coupled to any suitable actuation mechanism, such as a trigger-actuated tensioning mechanism (not shown). In embodiments, the conductor 4b may be configured as the pull-wire 107 and/or may be coupled to the pull-wire 107 allowing the pull-wire 107 to act as the conductor for the electrode 110.

With reference to Figs. 1 and 4A, the instrument 12 also includes one or more activation switches 150a, 150b, 150c, each of which extends through top-half shell portion of housing 100. Each activation switch 150a, 150b, 150c is operatively supported on a respective tactile element (e.g., a snap-dome switch) provided on a switch plate 154. Each activation switch 150a, 150b, 150c controls the transmission of electrical energy supplied from generator 200 to the electrodes 108 and 110. The activation switches 150a-150c transmit control signals via a voltage divider network (VDN) or other circuit control means through control leads within the cable 4 to the generator 200. For the purposes herein, the term "voltage divider network" relates to any known form of resistive, capacitive or inductive switch closure (or the like) which determines the output voltage across a voltage source (e.g., one of two impedances) connected in series. A "voltage divider" as used herein relates to a number of resistors connected in series, which are provided with taps at certain points to make available a fixed or variable fraction of the applied voltage.

With reference to Fig. 1, the instrument 12 further includes a slide switch 158 slidingly supported on or within housing 100 in a guide channel 160 defined therein. The switch 158 may be configured to function as a slide potentiometer, sliding over and along VDN. The switch 158 has a first position at a proximal-most position (e.g., closest to cable 4) corresponding to 0 % or a relatively low polarization setting, a second position wherein the switch 158 is at a distal-most position corresponding to 100 % or a relatively high polarization setting. The switch 158 may be disposed in a plurality of intermediate positions wherein the switch 158 is at positions between the distal-most position and the proximal-most position corresponding to various intermediate polarization settings. As can be appreciated, the polarization settings from the proximal end to the distal end may be reversed, e.g., high to low. Activation switches 150a-150c and the switch 158 are described in further detail in a commonly-owned U.S. Patent No. 7,879,033, the entire contents of which are incorporated by reference herein.

Fig. 7 shows the system 10 for applying plasma to a patient "P." The return electrode pad 6 is coupled to the patient. The return electrode pad 6 may be disposed underneath the patient "P" such that the patient "P" rests on top thereof. In embodiments, the return electrode pad 6 may be coupled to the patient "P" with conductive hydrogels and/or adhesives. As shown in Fig. 7, the system 10 may also include monopolar and bipolar surgical instruments 11a, 11b, respectively, which may be energized by the generator 200 to treat tissue.

The return electrode pad 6 is coupled to the polarization controller 170, which is in turn coupled to the connector 254 (Fig. 2) of the generator 200 via the cable 8. In embodiments, two or more return electrode pads 6 may be coupled to the patient "P." A splitter (not shown) may be used to couple multiple return electrode pads 6 to the generator 200 (e.g., at the connector 254). The splitter may be coupled to the polarization controller 170 prior to being connected to the generator 200. In further embodiments, multiple polarization controllers 170 may be utilized to accommodate a plurality of return electrode pads 6. In embodiments, the polarization controller 170 may be disposed within the generator 200 and be coupled to the input of the return electrode pad 6 at the generator 200 (e.g., connector 254).

The polarization controller 170 includes a variable resistance 172, which may be adjusted to control the conductive coupling of the return electrode pad 6 to the generator 200. Based on the conductivity of the return electrode pad 6, the instrument 12 may be operated in polarized, non-polarized, or hybrid manner. In particular, with the variable resistance 172 being fully activated such that the return electrode pad 6 is not coupled to the generator 200, the instrument 2 operates in a non-polarized manner, with the electrodes 108, 110 being only coupled to the generator 200 and therefore, being energized. With the variable resistance 172 being fully deactivated such that the return electrode pad 6 is fully-coupled to the generator 200. In this instance, the electrodes 108, 110 of the instrument 12 in combination with the return electrode pad 6 are connected to the generator 200 allowing for the operation of the system 10 in polarized manner.

Variable resistance 172 may include a plurality of resistors having a predetermined resistance that may be switched in and out of the circuit using switching elements (e.g., relays, transistors, field effect transistors, etc.). In embodiments, the variable resistance 172 may also include a variable potentiometer controllable by an electromechanical actuator. In further embodiments, the variable resistance 172 may include voltage-controlled resistances such as one or more transistors operated in its linear region or other semiconductor-based, electrically-controlled variable resistances, such as PIN diodes. The variable resistance 172 may be adjusted in fixed increments or may be infinitely variable (e.g., limited by electrical/physical limitations of its constituent components) or combinations thereof. Switching resistance in variable manner allows for fine-tuning the polarization of the system 10 to achieve a desired electrosurgical effect (e.g., maintaining constant current or constant voltage through the return electrode pad 6 or the instrument 12). Switching the resistance in a fixed manner (e.g., switching between fully-connected or fully-disconnected) variable resistance 172, allows for switching between non-polarized or polarized configurations, respectively.

Resistance of the polarization controller 170 may be controlled either through the generator 200 and/or the instrument 12. With reference to Fig. 2, the screen 244 may be a touchscreen that allows for control of the outputs the connectors 256 and 258 as well as the resistance of the polarization controller 170. In embodiments, the screen 244 may be replaced and/or supplemented by other controls (e.g., keyboard, buttons, etc.). The screen 244 includes input buttons for adjusting the degree of polarization. This may be accomplished by a variety of control schemes, shown on the screen 244 as graphical user interface elements, such as a slidable bar, predefined increment buttons, text and/or number inputs, and combinations thereof. The polarization settings may be displayed as a percentage or any other suitable scale for conveying the degree of polarization. The polarization settings are used by the generator 200 to adjust the resistance of the polarization controller 170, namely, the variable resistance 172 as described above to achieve a desired degree of polarization of the plasma outputted by the instrument 12.

The instrument 12 may also control various properties of the plasma beam. The activation switches 150a-150c may be used to activate the generator 200 and/or to control the flow of ionizable media from the ionizable media source 16. The slide switch 158 is configured to adjust the resistance of the polarization controller 170, namely, the variable resistance 172 as described above to achieve a desired degree of polarization of the plasma outputted by the instrument 12.

In embodiments, additional input devices may be used such as foot switches or handheld keyboards and/or remotes. The input devices (e.g., activation switches 150a-150c) may be two-stage switches where upon activation of the first stage, ionizable media and RF energy are supplied to the instrument 12 at a sufficient level to prime the active plasma field within the lumen 106 to initiate non-therapeutic ionization. This enables the user to visualize the target tissue relative to the non-therapeutic ionized gas plume. The generator 200 may include a feedback control loop to ensure the pre-ionization level is achieved and maintained at minimum needed RF power. In embodiments, a single wave spike may be generated to maintain sufficient ionized field without over heating the plasma instrument by minimizing RMS power delivered to pre-ionization field. In further embodiments, trace amounts of substantially non-electronegative compositions may be added to improve visibility of the ionized gas. Suitable tracer compositions include compounds such as sodium, neon, xenon, combinations thereof, and the like.

The closure of the second stage of the switch increases RF power to therapeutic levels and simultaneously increases conductivity through the return electrode pad 6 thereby initiating targeted therapeutic results. In particular, activation of the second stage would decrease the resistivity of the variable resistance 172 as described above.

The present disclosure provides for a plasma electrosurgical system with variable polarization, which allows for real-time adjustment of the plasma beam, thereby allowing for achieving specific surgical effects. The system also allows for used of standard electrosurgical generators (e.g., non-resonance matching generators operating in the radio frequency range at about 400 kHz) as a power source for exciting the plasma. Thus, a single electrosurgical generator may be used for generating plasma as well as operating with conventional electrosurgical instruments (e.g., monopolar, bipolar, etc.), thereby reducing the cost of operating room equipment.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure. In particular, as discussed above this allows the tailoring of the relative populations of plasma species to meet needs for the specific process desired on the workpiece surface or in the volume of the reactive plasma.

## Claims

1. A plasma instrument comprising:
an elongated body defining a first lumen therethrough, the lumen being in fluid communication with an ionizable media source;
an applicator tip coupled to a distal end of the elongated body and disposed within the first lumen, the applicator tip defining a second lumen in fluid communication with the first lumen;
a first electrode disposed on an outer surface of the applicator tip; and
a second electrode disposed within at least one of the first lumen or the second lumen, wherein the first and second electrodes are configured to be energized to ignite ionizable media supplied by the ionizable media source.

2. The plasma instrument according to claim 1, wherein the first electrode includes at least one of a plurality of concentric rings, a spiral, a plurality of interconnected strips, or a plurality of plates.

3. The plasma instrument according to claim 1 or 2, wherein the elongated body includes a shaft housing enclosing a return lead coupled to the first electrode.

4. The plasma instrument according to claim 3, wherein the shaft housing is flexible and the return lead is selectively tensionable to articulate the elongated body from a first generally relaxed position wherein proximal and distal portions of the elongated body are substantially aligned with a longitudinal axis defined by the elongated body to a second retracted position wherein the distal portion of the elongated body deflects from the longitudinal axis at a desired angle.

5. The plasma instrument according to any preceding claim, wherein the elongated body includes shaft housing having a conductive sheath disposed therein.

6. The plasma instrument according to claim 5, wherein the conductive sheath is in contact with the first electrode.

7. The plasma instrument according to claim 5 or 6, wherein the elongated body includes an insulative sheath disposed within the shaft housing such that the conductive sheath is disposed between the shaft housing and the insulative sheath.

8. The plasma instrument according to any preceding claim, wherein the first electrode includes an insulative layer and is supported within at least one of the first lumen or the second lumen by a spacer.

9. A plasma system comprising:
an electrosurgical generator;
an ionizable media source;
a plasma instrument comprising:
an elongated body defining a first lumen therethrough, the lumen being in fluid communication with the ionizable media source;
an applicator tip coupled to a distal end of the elongated body and disposed within the first lumen, the applicator tip defining a second lumen in fluid communication with the first lumen;
a first electrode disposed on an outer surface of the applicator tip; and
a second electrode disposed within at least one of the first lumen or the second lumen, wherein the first and second electrodes are coupled to the electrosurgical generator;
a return electrode pad configured to electrically couple to a patient; and
a polarization controller electrically coupled to the return electrode pad, the polarization controller configured to adjust conductive coupling of the return electrode pad to the electrosurgical generator.

10. The plasma system according to claim 9, wherein the polarization controller comprises a variable resistance.

11. The plasma system according to claim 10, wherein the variable resistance comprises a plurality of resistors coupled to a plurality of switching elements configured to switch the plurality of resistors into the variable resistance.

12. The plasma system according to claim 10, wherein the variable resistance comprises a variable potentiometer controllable by an electromechanical actuator.

13. The plasma system according to claim 10, wherein the variable resistance comprises a voltage-controlled resistance selected from the group consisting of a transistor, a PIN diode, and combinations thereof.

14. The plasma system according to claim 10, 11, 12 or 13, wherein at least one of the electrosurgical generator or the plasma instrument comprises controls for adjusting resistance of the polarization controller.

15. The plasma system according to any one of claims 9 to 14, wherein the plasma instrument is according to any one of claims 1 to 8.
